Europäisches Patentamt

European Patent Office

Office européen des brevets

(11) Publication number: **0 349 222**
**A2**

## EUROPEAN PATENT APPLICATION

(21) Application number: 89306416.2

(51) Int. Cl.⁴: **A61F 13/32**

(22) Date of filing: 23.06.89

(30) Priority: 30.06.88 GB 8815568
02.11.88 GB 8825631

(43) Date of publication of application:
03.01.90 Bulletin 90/01

(84) Designated Contracting States:
BE DE ES FR IT SE

(71) Applicant: **TAMBRANDS LIMITED**
**Dunsbury Way**
**Havant Hampshire, PO9 5DG(GB)**

(72) Inventor: **Akhter, Lala**
**2 Elm Tree Cottages**
**Prinsted Emsworth(GB)**
Inventor: **Browning, John Harold Douglas**
**1 Paddock Way**
**Alresford Hampshire, SO24 9PN(GB)**
Inventor: **Crease, Gary John**
**23 Bath Road**
**Southsea Portsmouth, PO4 0HX(GB)**
Inventor: **Guest, Malcolm**
**1 Down End Road**
**Drayton Portsmouth, PO6 1HT(GB)**

(74) Representative: **Alexander, Thomas Bruce et al**
**Boult, Wade & Tennant 27 Furnival Street**
**London EC4A 1PQ(GB)**

(54) Improvement in 2-piece tampon applicators.

(57) The present invention relates to a tampon applicator comprising an inner tube (12,52) for storing a tampon, an outer tube (2,42) slidably disposed over the inner tube (12,52) and having a distal discharge end (15,43) for insertion into the body of the user and means for resisting movement of the tampon towards the proximal end (4,49) when the inner tube (12,52) is partially withdrawn from the outer tube (2,42) further comprises means (6,46) engageable with the inner tube (12,52) for limiting sliding movement of the inner tube (12,52) and resisting complete withdrawal from the outer tube (2,42). The inner and outer tubes (12,52 and 2,42) are each formed from a paper or cardboard material and the limiting means (6,46) comprises at least one projection formed by cutting and/or by punching the material of the outer tube (2,42).

FIG. 8.

## IMPROVEMENTS IN 2-PIECE TAMPON APPLICATORS

This invention relates to tampon applicators for placing sanitary tampons in position within the body of a user of the tampon.

It is known to provide applicators in which an outer tube for storing a tampon therein has a distal discharge end for insertion into the body and the tampon is ejected by slidably moving an inner tube within the outer tube.

It has been convenient to make such applicators of paper or cardboard for ease of disposal and such applicators have been supplied in an initial condition in which the inner tube is only partially inserted into the outer tube in readiness for a single stroke dispensing operation. Consequently such applicators are relatively bulky.

In order to provide less bulky applicators a number of arrangements have been proposed in which the inner tube is initially received telescopically within the outer tube and must be partially withdrawn before the dispensing operation. Such applicators necessarily include some means of preventing the tampon being withdrawn from the outer tube with the inner tube as the inner tube is withdrawn.

An example of such an applicator is shown in GB 2120945 in which an applicator formed from a plastics material includes a projection formed on the inside of the outer tube for preventing withdrawal of the tampon while the inner tube is withdrawn.

However none of the proposals for applicators of this "compact" type in which the inner and outer tubes are initially provided one within the other has suggested an arrangement which may readily be formed from paper or cardboard material.

In accordance with the present invention there is disclosed a tampon applicator comprising an inner tube for storing a tampon therein, an outer tube slidably disposed over the inner tube and having a distal discharge end for insertion into the body of a user and means for resisting movement of the tampon towards the proximal end of the outer tube when the inner tube is partially withdrawn from the outer tube, which outer tube further comprises means engageable with the inner tube for limiting the sliding movement of the inner tube and resisting complete withdrawal of the inner tube from the outer tube, wherein the inner and outer tubes are each formed from a paper or cardboard material and the limiting means comprises at least one projection formed by cutting and/or punching the material of the outer tube.

An advantage of such an applicator is that it can be supplied in compact form whilst retaining the advantages of paper or cardboard material allowing for ease of disposal. A further advantage is that the applicator may be formed from the minimum number of components i.e., two components, by a simple and economic process.

Preferably the inner tube comprises a first longitudinal slot extending along part of the length of the inner tube but spaced from its distal end and the limiting means comprises a first projection of the outer tube extending into the first slot. The extent of withdrawal of the inner tube is therefore limited by engagement between the first projection and the distal end of the slot which is spaced from the distal end of the inner tube so that the inner tube cannot be fully withdrawn.

Conveniently the first projection is formed by making a discontinuous cut in the side wall of the outer tube and folding the cut material about a line extending in a substantially circumferential direction between the end portions of the cut such that the cut material extends in a radially inward direction with respect to the outer tube.

Preferably a part of the first projection is wider as viewed in axial projection than the width of the first slot adjacent the distal end of the inner tube to thereby captively retain the inner tube. Such an arrangement tends to avoid the possibility of the inner tube being withdrawn in spite of the presence of the first projection as a result of excessive force being applied.

Preferably the inner tube further comprises a second longitudinal slot extending from its distal end along part of its length and the movement resisting means comprises a second projection of the outer tube extending through the second slot.

Conveniently the second projection is formed by making a discontinuous cut in the side wall of the outer tube and folding the cut material along a substantially axially extending line extending between the end portions of the cut such that the cut material projects substantially radially inwardly with respect to the outer tube.

Preferably the first projection is axially spaced from the second projection and is closer to the proximal end of the outer tube.

Preferably, the second longitudinal slot narrows toward the proximal end of the inner tube.

The movement resisting means for the tampon may comprise the inter-engagement of a distal end portion of the tampon with the open distal end of the outer tube.

Preferably the first longitudinal slot has a flared portion adjacent its proximal end.

Preferably the flared portion includes a proximal end portion of reduced width, a shoulder being defined between said end portion and a wider part

 EP 0 349 222 A2  4

of the flared portion to avoid premature folding of the first projection if the inner tube is initially pushed inwards rather than pulled outwards from the outer tube.

Advantageously the outer surface of the outer tube may be coated to provide increased lubricity of the outer tube.

The distal end of the outer tube may be formed with a plurality of petal sections shaped to form a rounded distal end of the outer tube and is sufficiently flexible to allow passage of a tampon therethrough.

The inner tube may conveniently comprise indicator means for providing a visual indication of the position of movement of the inner tube relative to the outer tube in which the inner tube has been sufficiently withdrawn from the outer tube. The indicator means may be in the form of an indentation or other visible marking formed on the inner tube and positioned such that it becomes visible only when the inner tube has been withdrawn to the extent necessary for the distal end of the inner tube to be clear of the proximal end of a tampon contained in the applicator.

In a further preferred embodiment the limiting means also acts as the means for resisting movement of the tampon.

Preferably, the inner tube has a single longitudinal slot extending from its distal end to a point spaced from its proximal end and the limiting means extends into the slot.

Preferably, the longitudinal slot is stepped at the distal end of the inner tube providing an abutment for the limiting means.

Preferably, the longitudinal slot widens in the central region of the inner tube.

In a preferred embodiment the limiting means is formed by making a discontinous cut in the side wall of the outer tube and folding the cut material along a substantially axially extending line extending between the end portions of the cut such that the cut material projects substantially radially inwardly with respect to the outer tube. In a further preferred embodiment, the limiting means is formed by making a substantially Z-shaped cut in the side wall of the outer tube, the upper and lower strokes of the Z-shaped cut running circumferentially around part of the outer tube, and folding the cut material inwards about an axis which bisects the Z-shaped cut and which is substantially parallel to the tube axis.

Preferably, the outer tube is scalloped at its proximal and to facilitate removal of the tube.

In accordance with a further aspect of the present invention there is disclosed a sanitary tampon comprising an applicator and a tampon of compressed absorbent material within the inner tube, the tampon being adapted to expand radially when the inner tube is withdrawn from the outer tube to its proximal end position and being ejected from the outer tube through the distal end thereof when the inner tube is slidably returned to its distal end position.

Embodiments of the present invention will now be described by way of example only and with reference to the accompanying drawings of which:-

Figure 1 shows an exploded perspective view of a first embodiment of an applicator in accordance with the present invention;

Figure 2 is a sectional elevation of a sanitary tampon comprising the applicator of Figure 1 and a tampon;

Figure 3 is a sectional view taken at 3-3 in Figure 2;

Figure 4 is a sectional elevation showing the distal end portion of an alternative sanitary tampon having a rounded distal end;

Figure 5 is a perspective view of the distal end portion shown in Figure 4; and

Figure 6 is a view similar to Figure 2 of a modified sanitary tampon.

Figure 7 shows a further embodiment of the inner tube in Figure 1 with a modified slot 16.

Figure 8 shows an exploded perspective view of a further preferred embodiment of an applicator according to the present invention.

Figure 9 is a sectional elevation of the tampon applicator of Figure 8 with a sanitary tampon.

Figure 10 is a view of a second preferred embodiment of the outer tube of the applicator according to the present invention.

Figure 11 is a sectional elevation of the tampon applicator of Figure 10 with a sanitary tampon.

Figure 12 is a view of a further preferred embodiment of the outer tube of the applicator according to the present invention.

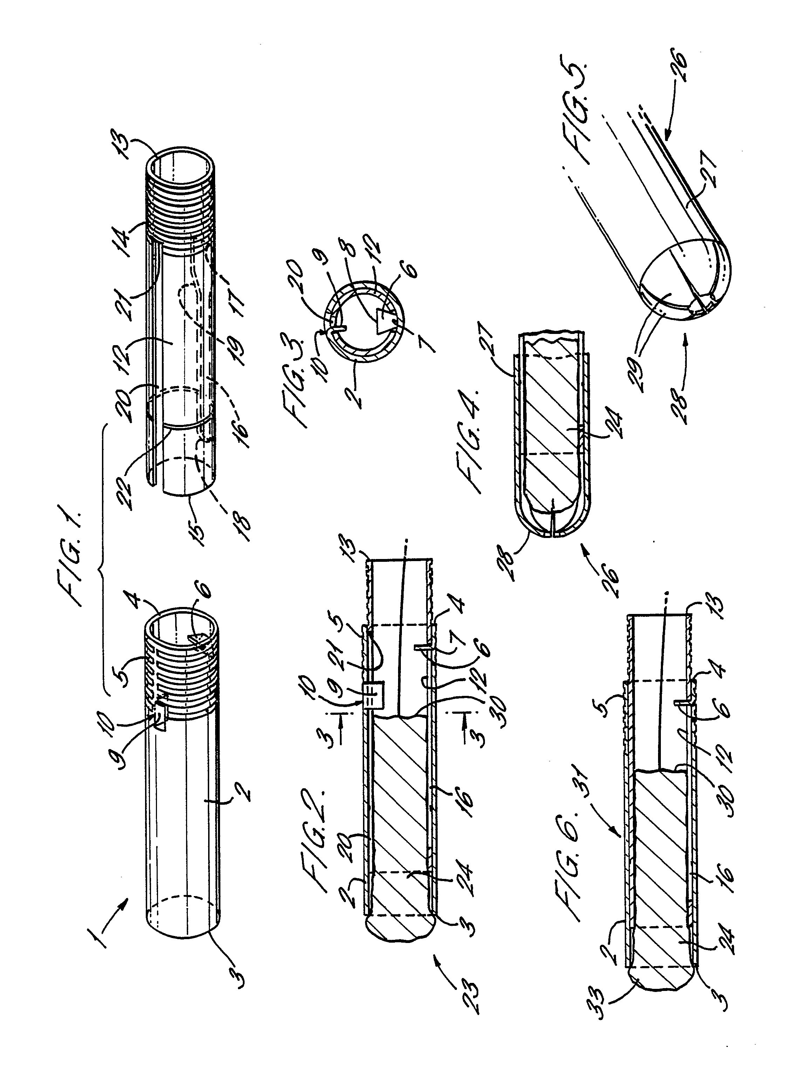

Referring first to Figures 1 to 3, an applicator 1 comprises an outer tube 2 of cardboard material formed by wrapped layers of paper bonded together with a suitable adhesive.

The outer tube 2 has an open distal end 3 and an open proximal end 4.

A series of six circumferential indentations 5 are axially spaced from one another and located adjacent to the proximal end 4 and are provided to facilitate gripping of the proximal end by a user.

A first projection 6 extends radially inwardly of the outer tube 2 at a location adjacent to the proximal end 4. The first projection 6 comprises part of the side wall of the outer tube which is cut around three sides of the projection and then bent radially inwardly about a fold line 7 to form a projection which is trapezoidal in shape having a free end 8 extending parallel to and of greater length than the fold line 7.

A second projection 9 extends radially inwardly of the outer tube 2 at a location adjacent to but distally spaced from the first projection 6 and diametrically opposed with respect to the outer tube 2. The second projection 9 is rectangular in shape and is also formed by a portion of the outer tube 2 which is cut around three sides and is bent inwardly about a fold line 10 extending axially with respect to the outer tube 2.

The applicator 1 further comprises an inner tube 12 which is slidably received within the outer tube 2 and is of greater length.

The inner tube 12 has a proximal end 13 adjacent to which are formed a series of six circumferential indentations 14 to assist gripping by a user.

The inner tube 12 has an open distal end 15. A first slot 16 extends axially along part of the legnth of the inner tube 12 and has a proximal end 17 which is axially spaced from the proximal end 13 of the inner tube 12, a distal end 18 of the first slot similarly being axially spaced from the distal end 15.

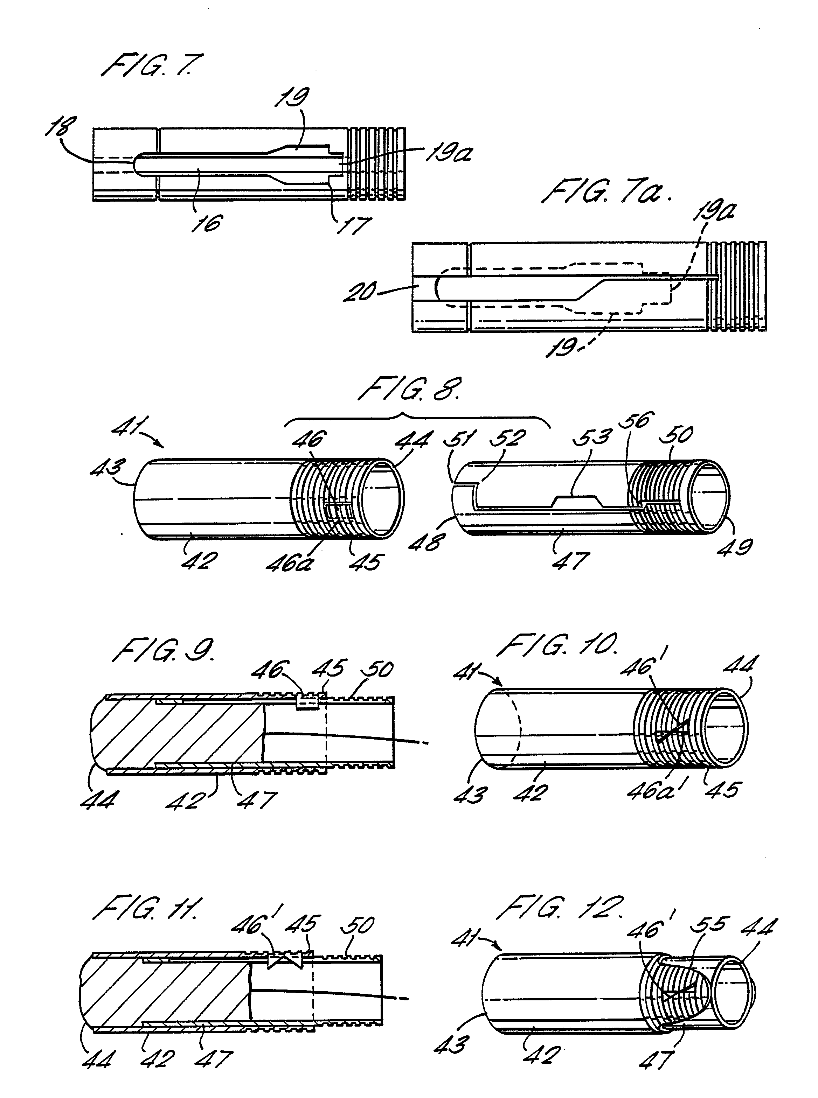

The first slot 16 includes a flared portion 19 adjacent its proximal end 17 which is of greater width than the free end 8 of the first projection 6, the remainder of the first slot being greater in width than the fold line 7 but narrower than the free end 8. The first projection 6 normally extends into the first slot 16 as shown in the assembled view of Figure 2 and as seen in Figure 3.

The inner tube 12 also includes a second slot 20 extending longitudinally from the distal end 15 of the inner tube to a proximal end location 21 which is axially spaced from the proximal end 17 of the first slot 16. The second slot 20 is diametrically opposite the first slot 16.

The second projection 9 normally extends through the second slot 20 as shown in the assembled views of Figures 2 and 3 and is of sufficient length to project radially inwardly with respect to the inner tube 12.

A circumferential groove 22 is provided on the external surface of the inner tube 12 towards its distal end 15 and adjacent to the distal end 18 of the first slot 16.

In Figure 2 a sanitary tampon 23 is shown to comprise the applicator 1 of Figure 1 together with a tampon 24 of compressed absorbent material located within the inner tube 12. The sanitary tampon 23 is shown in Figure 2 in its compact condition in which such a tampon would normally be stored and in which the inner tube 12 is compactly inserted within the outer tube 2 to the position shown in Figure 2 at which the first projection 6 abuts the proximal end 17 of the first slot 16.

Figures 4 and 5 illustrate the distal end of an alternative sanitary tampon 26 in which the outer tube 27 has a rounded distal end 28 comprising petal sections 29, the petal sections being sufficiently flexible to allow passage of the tampon 24 therethrough. The proximal end portion of the tampon assembly 26 is the same as tampon assembly 23 shown in Figures 1 to 3.

In use the sanitary tampon 23 of Figures 2 and 3 and including the applicator of Figure 1 is supplied to the user in the compact form described above, with reference to and as shown in Figure 2. The user first withdraws the inner tube 12 from the outer tube 2 to an extent sufficient for the distal end of inner tube 12 to clear the proximal end of tampon 24. This movement is limited by engagement of the first projection 6 with the distal end 18 of the first slot 16 which prevents complete withdrawal of the inner tube 12 from the outer tube 2. When the inner tube has reached its withdrawn position (not shown in the drawings) the circumferential groove 22 provides a visible indication to the user that the correct limit of withdrawal has been reached. During this withdrawal movement, proximal movement of the tampon 24 is resisted by action of the second projection 9 which abuts against a proximal end 30 of the tampon. In the open-end embodiment of Figures 1 to 3, movement of the tampon to the right as shown in Figure 2 will also be resisted by the engagement of the distal end of the tampon 24 with the open distal end of outer tube 2 as shown in Figure 2. However, in the closed-end embodiment of Figures 4 and 5, the second projection 9 will be the only substantial resistance to proximal movement of the tampon. The second projection also serves to prevent elongation of the tampon 24 as the inner tube 12 is withdrawn.

The sanitary tampon 23 is then primed for use and the distal end 3 of the outer tube 2 is inserted into the body whilst the user grips the proximal end 4 assisted by the indentations 5. The inner tube 12 is then advanced into the outer tube 2 by user manipulation of the proximal end 13 aided by the gripping indentations 14 and insertion is continued until the tampon 24 has been fully expelled from the outer tube 2.

Generally, the limit of this expelling movement will be when the proximal end of the inner tube 12 has been pushed to a position flush with the proximal end of the outer tube 2. During this movement, as the inner tube 12 passes the position shown in Figure 2 relative to the outer tube 2, the proximal end of first slot 16 will engage the first projection 6 and cause the first projection to fold flat thereby allowing the inner tube to continue its movement. It will be seen from Figure 2 that second slot 20 is sufficiently long to allow the full movement of inner tube 12 described above.

During this insertion of the inner tube 12 the distal end 15 of the inner tube engages the proximal end 30 of the tampon 24 such that the tampon is pushed axially so as to emerge from the distal end 3 of the outer tube 2. The applicator 1 is then withdrawn by the user for disposal.

Another function of the second projection 9 is to ensure that the inner tube 12 is withdrawn with the outer tube 2 on withdrawal of the applicator from the body.

It will be appreciated that because of the tendency of the tampon 24 to expand radially once the inner tube 12 is withdrawn to the extent that it no longer surrounds the tampon, the distal end 15 of the inner tube will abut the proximal end 30 of the tampon since it will have a smaller diameter. This difference in diameter may be enhanced by the tendency of the inner tube 12 to relax to a smaller diameter in the absence of the outward pressure of the compressed tampon material.

The alternative sanitary tampon 26 as shown in Figures 4 and 5 operates in a similar manner but accompanied by outward flexing of the petals 29 during ejection of the tampon 24. After ejection has been completed the petals 29 will remain deflected with the inner tube 12 projecting through them as withdrawal of the applicator takes place.

It will be appreciated that modifications may be made to the devices described above. Alternative arrangements are possible in which for example the groove 22 is replaced by a printed marking or other visible means of indicating the correct extent of withdrawal of the inner tube.

The indentations 5, 14, may be greater or less in number or may be replaced by small ribs or other suitable means to facilitate gripping of the tube by a user.

One or both of the projections 6, 9 may alternatively be formed by a punching operation instead of a cut-and-bend operation. A star shaped punch for example may be used to form an inward projection sufficient to co-operate with a co-operating slot of the inner tube.

In a further modification of the open-end tampon applicator, the second projection 9 and slot 20 may be omitted altogether. This modification is illustrated in Figure 6 in which like parts have the same reference numerals as in Figure 2. It will be seen that the modified tampon applicator 31 has inner and outer tubes similar to those in Figure 2 and includes a first projection 6 and slot 16. However, the second projection 9 is omitted. As slot 20 has been omitted, slot 16 is cut to the distal end. This short section of the slot is necessarily much narrower than the width of the long section of slot 16. In use of the tampon 31, as the inner tube 12 is withdrawn (moved to the right as shown in Figure 6) from the outer tube 2, proximal movement of the tampon 24 is resisted by engagement of an enlarged distal head portion 33 of the tampon with the open distal end 3 of the outer tube 2. The enlarged head portion of the tampon may be provided by shaping the tampon such that its distal end is wider than the open distal end 3 of the outer tube 2. Since the tampon material within the tubes is compressed, this may be achieved simply by use of a tampon of sufficient length to project distally from the outer tube 2 thereby naturally resuming an expanded diameter sufficient to prevent any proximal movement during withdrawal of the inner tube 12.

In another modification of the tampon applicator (not shown), the second projection 9 only may be omitted. In this embodiment the inner and outer tubes include a first projection 6, a slot 16 extending axially along part of the length of the inner tube 12 only and a slot 20.

In yet a further modification of the tampon applicator the first longitudinal slot 16 may be extended as shown in Figure 7 so that the flared portion 19 of the slot narrows at the proximal end of the slot. The narrowed portion 19a ensures that, if the user mistakenly pushes the inner tube 12 into the outer tube 2 rather than first withdrawing the inner tube 12 to prime the applicator for use, there will be no premature folding of the first projection 6.

As described earlier the first projection 6 extend inwards through the flared portion 19 of the slot 16 in the inner tube 12. If in this assembled state the inner tube 12 is pushed forwards into the outer tube (rather than pulled out in order to prepare the applicator for use) the projection 6 can be folded outwards and becomes disengaged from the slot 16. This makes the applicator ineffective because the inner tube 12 can be withdrawn completely from the outer tube 2 during preparation for use.

This premature folding of projection 6 can be avoided if there is a rearwards extension 19a to the slot 16 in the inner tube 12 and the projection 6 can be located within the extension. The width of the extension 19a must be less than the maximum width of the projection 6 (which as shown in Figures 1 and 3 is a trapezoidal shape). In this way, any pushing of the inner tube 12 into the outer tube 2 before preparation for use will be resisted by the projection 6 which is retained in the slot extension 19a.

During manufacturing assembly of the applicator the flared part 19a of the slot 16 is located such that the projection 6 in the outer tube 2 can be folded into the flared portion 19 without deforming the shape of the projection 6. The inner tube 12 is then moved such that the projection 6 is located in the slot extension 19a. In this position, the projection 6 inhibits further toward movement of the inner tube 12 within the outer tube 2.

In use the applicator is prepared by pulling the inner tube 12 from the outer tube 2. The projection 6 rides under the slot 16 and provides a stop when the inner tube 12 is fully extended. When the tubes are subsequently pushed together to expel the tampon, the projection 6 is lifted out of the slot 16 when the base of the projection contacts the start of the slot extension 19a. The inner tube 12 can then be pushed fully into the outer tube 2 ensuring total expulsion of the tampon.

A further modification as shown in Fig. 7a involves the narrowing of the second longitudinal slot 20 towards the proximal end of the applicator. The advantage of such narrowing is that when the applicator is in the unprimed condition the second projection 9 is surrounded by solid tube rather than a hole which improves the outer appearance of the applicator.

Referring now to Figures 8 and 9, an applicator 41 comprises an outer tube 42 of cardboard material formed by wrapped layers of paper bonded together with a suitable adhesive. The outer tube 42 has an open distal end 43 and an open proximal end 44.

A series of six circumferential indentations 45 are axially spaced from one another and located adjacent to the proximal end 44 and are provided to facilitate gripping of the proximal end by a user.

A projection 46 extends radially inwardly of the outer tube 42 at a location adjacent the proximal end 44. The projection 46 comprises part of the side wall of the outer tube which is cut around three sides of the projection and then folded radially inwardly about a fold line 46a extending axially with respect to the outer tube.

The applicator 41 further comprises an inner tube 47 which is slidably received within the outer tube 42. The inner tube 47 has a proximal end 49 and a distal end 48.

The proximal end 49 has a series of six circumferential indentations 50 to assist gripping by a user.

A slot 51 extends axially along the inner tube from the distal end 48 to a point which is spaced from the proximal end 49 of the inner tube 47. The slot 51 has a stepped portion 52 adjacent the distal end 48 of the inner tube 47, a portion 53 which is of greater width and a further stepped portion 56 adjacent the proximal end of the inner tube 47. When the applicator 41 is assembled the inner tube 47 is pushed into the outer tube 42 until the projection 46 lies over the widened portion 53 of slot 51. The projection 46 is then folded inwards and the inner tube 47 is then pushed into the outer tube 42 until the projection 46 abuts the stepped portion 56 of slot 51. A tampon 54 of compressed absorbent material can then be inserted into the distal end of the inner tube 47.

Figures 10-12 illustrate alternative embodiments of the present invention.

Figures 10 and 11 show a modification of the outer tube 42 in which like parts have the same reference numerals as in Figure 8. It will be seen that the modified outer tube has a projection 46′ formed by cutting a Z-shape in the side wall. The Z-shape is then folded inwardly about fold line 46a′ to produce two triangular flaps. The outer tube 42 depicted in Figures 10 and 11 can be used with the inner tube 47 depicted in Figure 8. The applicator 41 is then assembled in a similar manner to that described with reference to Figures 8 and 9. The Z-shaped cut which forms projection 46′ ensures that the inner tube 47 cannot be rotated with respect to the outer tube 42 thus preventing the projection 46′ from rotating out of the slot 51. Clearly, the projection 46 depicted in Figures 8 and 9 does not prevent rotation of the inner tube 47 with respect to the outer tube 42 and thus is able to rotate out of slot 51 and allow the inner tube 47 to be completely withdrawn from the outer tube 42.

Figure 12 shows a further modification to the outer tube 42. Again, like parts have the same reference numerals as in Figures 8-11. It will be seen that the proximal end 44 of the outer tube 42 has been cut away to provide scalloped portions 55. The scalloped portions 55 allow the inner tube 47 to be pushed completely within the outer tube 42 whilst still allowing the user to withdraw the inner tube. In this way, a compact applicator is provided which can be reduced in length by the amount which was previously necessary to ensure that the inner tube 47 could be gripped to withdraw it from the outer tube 42. The inner tube 47 in Figure 12 is identical to that shown in Figure 8 but shorter.

In use the tampon applicator is supplied to the user in compact form as shown, for example, in Figures 9 and 11. The user first withdraws the inner tube 47 from the outer tube 42 until the projection 46 abuts the distal end of slot 51 at step 52. The projection 46 not only limits the movement of the inner tube 47 with respect to the outer tube 42 to prevent complete withdrawal but also partially prevents tampon 54 from being withdrawn with the inner tube 47. When the projection 46 abuts the distal end of slot 51 at step 52 the sanitary tampon 54 is then primed for use and the distal end 43 of the outer tube 42 is inserted into the body whilst the user grips the proximal end 44 assisted by indentations 45. The inner tube 47 is then pushed into the outer tube 42 and insertion is continued until tampon 54 is expelled fully from the outer tube 42. The applicator 41 is then withdrawn by the user for disposal.

Another function of projection 46 is to ensure that the inner tube 47 is withdrawn with the outer tube 42 on withdrawal of the applicator from the body.

It will be appreciated that because of the tendency of the tampon 54 to expand radially once the inner tube 47 is withdrawn to the extent that it no longer surrounds the tampon, the distal end 48 of the inner tube 47 will abut the proximal end of the tampon 54 since it will have a smaller diameter. This difference in diameter may be enhanced by the tendency of the inner tube to relax to a smaller diameter in the absence of the outward pressure of the compressed tampon material.

It will be appreciated that modifications may be made to the devices described above.

The indentations 45, 50 may be greater or less in number or may be replaced by small ribs or other suitable means to facilitate gripping of the tube by a user.

The scalloped portions 55 in the outer tube may be shaped differently to facilitate gripping of the inner tube 47 with respect to the outer tube 42.

The projections 46, 46' may alternatively be formed by a punching operation instead of a cut-and-bend operation.

## Claims

1. A tampon applicator comprising an inner tube for storing a tampon therein, an outer tube slidably disposed over the inner tube and having a distal discharge end for insertion into the body of a user and means for resisting movement of the tampon towards the proximal end of the outer tube when the inner tube is partially withdrawn from the outer tube, which outer tube further comprises means engageable with the inner tube for limiting the sliding movement of the inner tube and resisting complete withdrwal of the inner tube from the outer tube, wherein the inner and outer tubes are each formed from a paper or cardboard material and the limiting means comprises at least one projection formed by cutting and/or punching the material of the outer tube.

2. An applicator as claimed in claim 1 in which the inner tube comprises a first longitudinal slot extending along part of the length of the inner tube but spaced from its distal end and the limiting means comprises a first projection of the outer tube extending into the first slot.

3. An applicator as claimed in claim 2 in which the first projection is formed by making a discontinous cut in the side wall of the outer tube and folding the cut material about a line extending in a substantially circumferential direction between the end portions of the cut such that the cut material extends in a substantially radially inward direction with respect to the outer tube.

4. An applicator as claimed in claim 2 or claims 3 in which a part of the first projection is wider as viewed in axial projection than the width of the first slot adjacent the distal and of the inner tube to thereby captively retain the inner tube.

5. An applicator as claimed in any preceding claim in which the inner tube further comprises a second longitudinal slot extending from its distal end along part of its length and the movement resisting means comprises a second projection of the outer tube extending through the second slot.

6. An applicator as claimed in claim 5 in which the second projection is formed by making a discontinuous cut in the side wall of the outer tube and folding the cut material along a substantially axially extending line extending between the end portions of the cut such that the cut material projects substantially radially inwardly with respect to the outer tube.

7. An applicator as claimed in claim 5 or claim 6 in which the first projection is axially spaced from the second projection and is closer to the proximal end of the outer tube.

8. An applicator as claimed in any of Claims 5, 6 or 7 wherein the second longitudinal slot narrows towards the proximal end of the inner tube.

9. An applicator as claimed in any of claims 1 to 4 in which the movement resisting means for the tampon comprises the inter-engagement of a distal end portion of the tampon with the open distal end of the outer tube.

10. An applicator as claimed in any preceding claim in which the first longitudinal slot has a flared portion adjacent its proximal end.

11. An applicator as claimed in claim 10 in which the flared portion includes a proximal end portion of reduced width, a shoulder being defined between said end portion and a wider part of the flared portion.

12. An applicator as claimed in any preceding claim in which the outer surface of the outer tube is coated to provide increased lubricity of the outer tube.

13. An applicator as claimed in any preceding claim in which the distal end of the outer tube is formed with a plurality of petal sections shaped to form a rounded distal end of the outer tube and sufficiently flexible to allow passage of a tampon therethrough.

14. An applicator as claimed in any preceding claim in which the inner tube further comprises indicator means for providing a visual indication of the portion of movement of the inner tube relative to the outer tube in which the inner tube has been sufficiently withdrawn from the outer tube.

15. An applicator as claimed in claim 1 in which the limiting means also acts as the means for resisting movement of the tampon.

16. An applicator as claimed in claim 15 in which the inner tube has a single longitudinal slot extending from its distal end to a point spaced from its proximal end and the limiting means extends into the slot.

17. An applicator as claimed in claim 16 in which the longitudinal slot is stepped at the distal end of the inner tube providing an abutment for the limiting means.

18. An applicator as claimed in claim 16 or claim 17 in which the longitudinal slot widens in the central region of the inner tube.

19. An applicator as claimed in any of claims 15 to 18 in which the limiting means is formed by making a discontinuous cut in the side wall of the outer tube and folding the cut material along a substantially axially extending line extending between the end portions of the cut such that the cut material projects substantially radially inwardly with respect to the outer tube.

20. An applicator as claimed in any of claims 15 to 18 in which the limiting means is formed by making a substantially Z-shaped cut in the side wall of the outer tube, the upper and lower strokes of the Z-shaped cut running circumferentiually around part of the outer tube, and folding the cut material inwards about an axis which bisects the Z-shaped cut and which is substantially parallel to the tube axis.

21. An applicator as claimed in any preceding claim in which the outer tube is scalloped at its proximal end to facilitate withdrawal of the inner tube.

22. A sanitary tampon comprising an applicator as claimed in any of the preceding claims and a tampon of compressed absorbent material within the inner tube, the tampon being adapted to expand radially when the inner tube is withdrawn from the outer tube to its proximal end position and being ejected from the outer tube through the distal end thereof when the inner tube is slidably returned to its distal end position.

23. An applicator substantially as hereinbefoire described with reference to and as shown in the accompanying drawings.

24. A sanitary tampon substantially as hereinbefore described with reference to and as shown in the accompanying drawings.

FIG.1.

FIG.2.

FIG.3.

FIG.4.

FIG.5.

FIG.6.

FIG. 7

FIG. 7a.

FIG. 8.

FIG. 9.

FIG. 10.

FIG. 11.

FIG. 12.